# EUROPÄISCHE PATENTSCHRIFT

(11) **EP 3 856 314 B1**
(45) Veröffentlichungstag und Bekanntmachung des Hinweises auf die Patenterteilung: **28.12.2022**
(21) Anmeldenummer: 19702059.7
(22) Anmeldetag: 24.01.2019
(51) Int. Cl.: A61M 16/20, A61M 16/08, A61M 16/16

(54) **LECKAGENVENTIL SOWIE BEATMUNGSSCHLAUCHSYSTEM**
LEAKAGE VALVE AND VENTILATION TUBE SYSTEM
VALVE À FUITE INTENTIONNELLE AINSI QUE SYSTÈME DE TUBES RESPIRATOIRES

(43) Veröffentlichungstag der Anmeldung: 04.08.2021
(73) Patentinhaber: Löwenstein Medical Technology S.A., 2557 Luxembourg (LU)
(72) Erfinder: KLINGER, Miriam, 91126 Schwabach (DE)
(74) Vertreter: Marx, Thomas
(86) Internationale Anmeldenummer: PCT/EP2019/051781
(87) Internationale Veröffentlichungsnummer: WO 2020/151827

(56) Entgegenhaltungen:
- EP-A2- 1 138 340
- EP-A2- 1 314 446
- WO-A1-2016/063168
- DE-B3-102007 052 898
- US-A- 5 937 851

## Beschreibung

Die vorliegende Erfindung betrifft ein Leckagenventil gemäß dem Oberbegriff des Anspruchs 1 sowie ein Beatmungsschlauchsystem gemäß Anspruch 10.

### Technologischer Hintergrund

Leckagenventile werden bei der sogenannten Leckagenbeatmung eingesetzt. Hierbei bekommt der zu beatmende Patient von einem Beatmungsgerät eine Menge an Beatmungsgas zur Verfügung gestellt, die größer ist als die vom Patienten bei der Atmung verbrauchte Menge an Beatmungsgas. Der Rest dieser vom Beatmungsgerät zur Verfügung gestellten Beatmungsgasmenge verlässt den Beatmungskreis über das Leckagenventil. Bei der Inspiration durchströmt das Beatmungsgas das Innenlumen des Leckagenventils und wäscht zugleich auch alle Reste an verbrauchter Luft aus dem Leckagenventil heraus. Das Beatmungsgerät besitzt eine exakt definierte Förderleistung an Beatmungsgas. Diese Förderleistung kann an die betreffenden patientenabhängigen Verbrauchsmengen (z.B. Beatmung eines Erwachsenen oder eines Säuglings) geräteseitig angepasst werden. Beatmungsgeräte zur Leckagenbeatmung sind üblicherweise genau auf die durch das Leckagenventil festgelegte Leckagenmenge abgestimmt. Sofern es zu Schwankungen der Leckagenmenge kommt, werden diese nicht erfasst und führen daher zu einer nicht gewünschten Beeinträchtigung der Beatmung, was insbesondere bei der Beatmung von Neugeborenen von besonderer Wichtigkeit ist.

### Druckschriftlicher Stand der Technik

Ein Leckagenventil gemäß dem Oberbegriff des Anspruchs 1 ist aus der EP 2 428 243 B1 bekannt. Die beiden Gehäuseteile besitzen eine gegenseitige Stufung sowie Durchtrittöffnungen im Bereich des radial nach außen vorspringenden Gehäusebereichs. Bei diesem bekannten Leckagenventil sollen die beiden miteinander verrastenden Gehäuseteile zur Vermeidung einer Verdrehung zueinander durch eine Klemmverbindung verbunden sein.

Weiterhin ist aus der US 5 937 851 ein Leckagenventil bekannt, welches ebenfalls aus einem Außenteil sowie einem damit verrastbaren Innenteil besteht. Das Innenteil verfügt über Federlaschen, die am stirnseitigen Ende des Außenteils angreifen. Das Außenteil ist zur Bildung einer umlaufenden spaltförmigen Drosselkammer zum Innenteil beabstandet und weist an dessen breiter Stirnseite drei schmale sowie eine breite Erhöhung auf, wobei die Erhöhungen zusammen mit dem Innenteil ein bereichsweises Ausströmen von CO₂ haltiger Atemluft aus der Drosselkammer ermöglichen. Zusätzlich können im Innenteil entfernt zu einem radial nach außen vorspringenden Gehäusebereich mehrere Durchtrittöffnungen für einen zusätzlichen Luftdurchtritt von der Innenseite des Leckagenventils in die Drosselkammer vorgesehen sein. Innenteil und Außenteil sollen gegeneinander verdrehbar sein. Bei dieser Konstruktion besteht das Problem, dass bei mechanischer Beeinträchtigung des Leckagenventils es zu erheblichen Schwankungen der Leckagenmenge kommen kann.

Aus EP 1 314 446 ist eine Beatmungsmaske bekannt, bei der ein Maskengrundkörper über ein Kupplungselement mit einem Ausatmungselement verbunden ist, wobei das Kupplungselement gemeinsam mit dem Ausatmungselement einen Ausströmkanal begrenzt. Das Ausatmungselement ist dazu bereichsweise in einen Halterungsstutzen des Kupplungselements eingeführt. Eine maximale Einführtiefe wird durch Außenstege vorgegeben, die im Wesentlichen parallel zu einer Längsachse des Ausatmungselements entlang einer Außenseite des Ausatmungselements verlaufen. Das Ausatmungselement weist außerdem entlang seines Umfangs mehrere radial sich erstreckende Verbindungsstege auf, zwischen denen Ausströmöffnungen vorgesehen sind. Die Verbindungsstege verlaufen mit ihrer Außenseite leicht nach innen. Die Ausströmöffnungen befinden sich oberhalb der Außenstege. Das Ausatmungselement ist innerhalb des Kupplungselementes von Arretierungselementen fixiert, die mit jeweils einer Rastnase einen zugeordneten Absatz hintergreifen. Ein Spiel zwischen Halterungsstutzen und Ausatmungselement kann eine einfache Drehbarkeit des Ausatmungselementes innerhalb des Halterungsstutzens unterstützen.

Die EP 1 138 340 A2 betrifft eine Vorrichtung zur Ableitung von zumindest teilweise verbrauchtem Atemgas aus einem Atemgasleitungssystem. Die Vorrichtung umfasst einen ersten Anschlussabschnitt und einen zweiten Anschlussabschnitt sowie eine Ableitungseinrichtung zur Ableitung eines Atemgasteilstromes, wobei die Ableitungseinrichtung durch wenigstens einen Kanal gebildet ist, der zumindest teilweise durch die Wandung eines elastomeren Körpers begrenzt ist. Die Kanäle befinden sich im Bereich eines radial nach außen vorragenden Wulstes. Zur drehbaren Koppelung der Anschlussabschnitte ist eine Drehmuffeneinrichtung vorgesehen. Bei einer bestimmten Ausführungsform können mehrere radial nachgiebige Rastzungenelemente vorgesehen sein, die lösbar mit einem rohrbuchsenartig ausgebildeten zweiten Anschlusselement in Eingriff bringbar sind.

Die DE 10 2007 052 898 B3 offenbart eine Vorrichtung zur Ableitung von Atemgas mit einem Außenteil und einem Innenteil, welches eine zylindrische Gleitfläche aufweist. Das Innenteil ist über ein Verbindungselement drehbar innerhalb des Außenteils befestigt. Zwischen dem Verbindungselement und dem Außenteil wird ein ringförmiger Ausströmspalt für Atemgas gebildet. Der Ausströmspalt wird durch Distanzelemente zwischen dem Verbindungselement und dem Außenteil gebildet. Die Distanzelemente sind derart ausgebildet, dass das Verbindungselement gegenüber dem Außenteil verdrehsicher fixiert ist. Die Distanzelemente weisen mit dem Außenteil verbindbare Rastzungen auf.

Aus WO 2016/063168 A1 ist ein einstellbares Leckventil bekannt, das konfiguriert ist, um ein Lecken während einer Atemtherapie zu steuern. Das Leckventil umfasst einen röhrenförmigen Körper, der einen Strömungsweg bildet, um während der Atemtherapie Gas zuzuführen, wobei der röhrenförmige Körper in der Wand gebildete Körperöffnungen aufweist, die Gas von dem Strömungsweg durch seine Wand leiten. Eine Muffe ist in Bezug auf den röhrenförmigen Körper um eine Achse drehbar, wobei die Muffe die Körperöffnungen in dem Körper im Wesentlichen umschließt. Die Muffe weist zudem Muffenöffnungen auf, die derart positionierbar sind, dass sie mit den Körperöffnungen überlappen. Ein röhrenförmiger Einstellring umschließt im Wesentlichen die Muffenöffnungen in der Muffe, wobei der Einstellring eine darin gebildete Einstellringöffnung aufweist. Die Einstellringöffnung ist derart positionierbar, um mit den Muffenöffnungen und den Körperöffnungen zu überlappen, wobei eine Drehkupplung zwischen dem röhrenförmigen Körper, der Muffe und dem Einstellring eine einstellbare Konfiguration einer oder mehrerer Eigenschaften einer Leckbahn vereinfacht, die durch Positionieren der Überlappung der Einstellringöffnung mit mindestens einer der Muffenöffnungen und mindestens einer der Körperöffnungen gebildet wird, um ein Lecken während der Atemtherapie zu erlauben.

### Aufgabe der vorliegenden Erfindung

Die Aufgabe der vorliegenden Erfindung besteht darin, ein gattungsgemäßes Leckagenventil zu schaffen, mittels dem Schwankungen der Leckagenmenge wirksam vermieden werden können.

### Lösung der Aufgabe

Die vorstehende Aufgabe wird durch ein Leckagenventil mit den Merkmalen des Anspruchs 1 gelöst.

Zweckmäßige Ausgestaltungen werden in den abhängigen Ansprüchen beansprucht.

Im Gegensatz zu dem Gegenstand der EP 2 428 243 B1 sind bei dem erfindungsgemäßen Leckagenventil die beiden Gehäuseteile im verrasteten Zustand zueinander verdrehbar. Dies hat den Vorteil, dass es zu keinen Verspannungen im Einsatz kommen kann, da sich die beiden Gehäuseteile beim Auftreten von mechanischen Beeinträchtigungen bzw. Torsionsbelastungen zueinander verdrehen, wodurch sich keine Spannungen, die zu einer mechanischen Beeinträchtigung führen, aufbauen können. Dies wiederum bewirkt, dass auf das Leckagenventil weit weniger mechanische Beeinträchtigungen einwirken können, die zu einer Schwankung der Leckagenmenge führen. Zudem zeichnet sich das erfindungsgemäße Leckagenventil durch eine herstellungstechnisch günstige Konstruktion aus.

Zweckmäßigerweise wird die Drehbarkeit der beiden Gehäuseteile zueinander dadurch erreicht, dass im verrasteten Zustand der beiden Gehäuseteile zwischen der axial verlaufenden Rastlaschen und der axial verlaufenden Innenwand des ersten Anschlussstutzens ein Spaltspiel vorgesehen ist.

Ebenso kann im verrasteten Zustand der beiden Gehäuseteile zwischen der stirnseitigen Nase der Rastlaschen und dem stirnseitigen Ende des ersten Anschlussstutzens ein Spaltspiel vorgesehen sein.

Ferner kann im verrasteten Zustand der beiden Gehäuseteile zwischen der axial verlaufenden Außenwand der gekrümmten Wandbereiche und der axial verlaufenden Innenwand des ersten Anschlussstutzens ein Spaltspiel vorgesehen sein.

Das Maß des Spalts (Spaltspiel) für die Drehbarkeit der beiden Gehäuseteile beträgt im Wesentlichen 0,08 mm bis 0,12 mm (d.h. auf den Durchmesser bezogen 0,04 mm bis 0,06 mm pro Seite), vorzugsweise 0,09 mm bis 0,11 mm (d.h. auf den Durchmesser bezogen 0,045 mm bis 0,055 mm pro Seite).

Die vorgenannte Konstruktion bewirkt, dass sich die beiden Gehäuseteile zueinander ohne Kraftaufwendung verdrehen können, hierdurch das Auftreten von mechanischen Spannungen auf das Leckagenventil während des Einsatzes vermieden werden kann und die Leckagenmenge keinerlei Schwankungen unterliegt.

Dadurch dass am zweiten Gehäuseteil entlang des Umfangs mehrere radial sich erstreckende Stege vorgesehen sind, die Durchgangsöffnungen begrenzen, wird ein besonders vorteilhafter, großflächiger Einströmbereich für die Leckagenströmung in den Leckagenkanal begründet. Gleichzeitig trägt die Konstruktion dazu bei, die Verdrehbarkeit der beiden Gehäuseteile zueinander zu erleichtern.

Zweckmäßigerweise weist der die Durchgangsöffnungen aufweisende Gehäuseteil, bis auf die radial nach außen ragenden Stege, keine Abstufung auf.

Dadurch dass die Stege den gekrümmten Wandbereich zur Außenseite hin überragen, ist es möglich, die Stege gleichzeitig als Auflager für das erste Gehäuseteil zu verwenden.

Erfindungsgemäß besteht zwischen der Innenwand des erweiterten Gehäusebereichs und dem außenseitigen Ende des jeweiligen Stegs ein umlaufender Ringraum. Hierdurch ist es möglich, dass Beatmungsgas auch um die Stirnseite des Stegs herum durch den dort befindlichen Ringraum und von dort in den Leckagenkanal nach außen strömen kann, wodurch die Leckagenmenge zusätzlich stabilisiert wird.

Zweckmäßigerweise liegt im verrasteten Zustand der beiden Gehäuseteile der erste Gehäuseteil auf den Stegen des zweiten Gehäuseteils, vorzugsweise stumpf, auf. Diese Konstruktion hat den Vorteil, dass bei Verdrehung der beiden Gehäuseteile zueinander konstruktionsbedingt eine nur sehr geringe Flächenreibung auftritt, wodurch sich eine hervorragende Verdrehbarkeit der beiden Gehäuseteile zueinander einstellt.

Zweckmäßigerweise weist das erste Gehäuseteil hierzu eine umlaufende Stufenfläche auf, die im verrasteten Zustand der beiden Gehäuseteile oberseitig auf den Stegen aufliegt.

Das erste und/oder zweite Gehäuseteil sind vorzugsweise einteilig ausgebildet.

Ferner kann das zweite Gehäuseteil im Bereich des erweiterten Gehäusebereichs eine ringartige Erweiterung bzw. einen ringartigen Vorsprung aufweisen, der sich radial nach außen erstreckt und zusammen mit der inneren Wandung des ersten Gehäuseteils den in das Freie führenden Leckagenkanal bildet.

Dadurch dass die ringartige Erweiterung an deren Unterseite mit einer umlaufenden Ausnehmung versehen ist, wird die mechanische Stabilität des zweiten Gehäuseteils insgesamt zusätzlich verstärkt.

Die vorliegende Erfindung betrifft des Weiteren ein Beatmungsschlauchsystem zur Verwendung bei einer Leckagenbeatmung, wobei das Beatmungsschlauchsystem einen vorzugsweise beheizbaren Beatmungsschlauch sowie ein Leckagenventil gemäß den Ansprüchen 1 bis 9 umfasst.

Das Beatmungsschlauchsystem zeichnet sich weiterhin darin aus, dass an mindestens einem Ende, vorzugsweise an beiden Enden des Beatmungsschlauchs, eine Anschlussmuffe zum Anschluss des Beatmungsschlauchs an das Leckagenventil und/oder an ein Funktionsteil vorgesehen ist.

Zweckmäßigerweise befindet sich das Leckagenventil zwischen der Anschlussmuffe und einer am Patienten positionierten Beatmungshilfe.

Zweckmäßigerweise befindet sich das Leckagenventil zwischen der Anschlussmuffe und einem Konnektor mit Sondenanschlussmittel.

### Beschreibung der Erfindung anhand von Ausführungsbeispielen

Nachstehend wird eine zweckmäßige Ausgestaltung des erfindungsgemäßen Leckagenventils im Detail beschrieben. Wiederkehrende Merkmale sind der Übersichtlichkeit halber lediglich einfach gekennzeichnet. Es zeigen:
- Fig. 1: eine stark vereinfachte schematische Darstellung eines Beatmungssystems zur Leckagenbeatmung;
- Fig. 2: eine Seitenansicht einer zweckmäßigen Ausgestaltung eines erfindungsgemäßen Leckagenventils;
- Fig. 3: eine Schnittdarstellung des Leckagenventils gemäß Fig. 2 entlang der Schnittlinie A-A;
- Fig. 4: eine Detailschnittdarstellung im Bereich Z von Fig. 3;
- Fig. 5: eine Draufsicht auf die Oberseite des Leckagenventils gemäß Fig. 2;
- Fig. 6: eine Schnittdarstellung entlang der gewinkelten Schnittflächen B-B von Fig. 5; sowie
- Fig. 7: eine Darstellung der Funktion bzw. des Zusammenbaus des erfindungsgemäßen Leckagenventils gemäß Fig. 2 in verrastetem Zustand (Fig. 7a), in einer Explosionsdarstellung (Fig. 7b) sowie ebenfalls in einer Explosionsdarstellung, jedoch um 90° gedreht zu Fig. 7b (Fig. 7c).

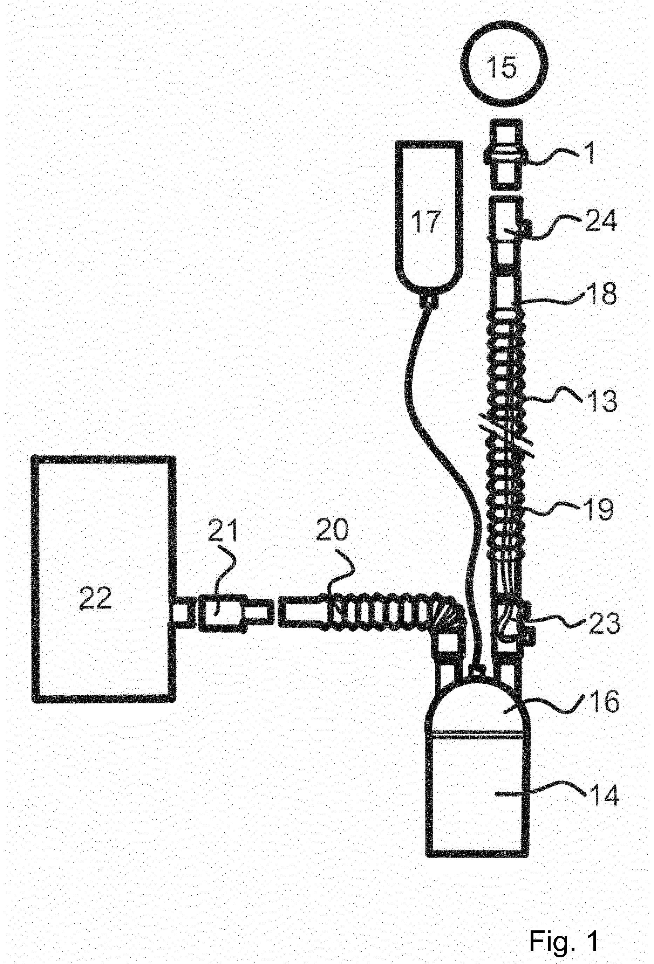

Fig. 1 zeigt ein Beispiel einer Anordnung zur Leckagenbeatmung. Bei der Bezugsziffer 15 handelt es sich um eine am Patienten befindliche Beatmungseinrichtung, wie z.B. eine Beatmungsmaske, die mit einem (nicht dargestellten) Anschlussstutzen versehen ist und mit einem Leckagenventil 1 gemäß der Erfindung verbunden wird. An das Leckagenventil 1 kann sich z.B. ein Konnektor 24 mit einem Anschluss für eine Sonde anschließen.

Des Weiteren ist ein Beatmungsschlauch 13 vorgesehen, welcher bei Bedarf einen Heizdraht beinhalten kann, der dazu vorgesehen ist, das Beatmungsgas innerhalb des Beatmungsschlauchs auf einer bestimmten Temperatur zu halten. Ein Heizdraht muss allerdings nicht unbedingt vorhanden sein. Der Beatmungsschlauch 13 weist an seinem jeweiligen Ende je eine Anschlussmuffe 18, 19 auf. Ferner steht der Beatmungsschlauch 13 über einen weiteren Konnektor 23 mit Anschluss für eine Sonde mit einer Befeuchterkammer 16 eines Luftbefeuchters 14 in Verbindung. In der Befeuchterkammer 16 wird speziell konditioniertes Beatmungsgas bereitgehalten. Die Befeuchterkammer 16 steht über einen Verbindungsschlauch 20 und einen Verbinder 21 mit einem Ventilator 22 in Verbindung. Um einen gewissen Feuchtegehalt des innerhalb der Befeuchterkammer 16 befindlichen Beatmungsgases sicherzustellen, steht diese mit einem Wasserreservoir 17 in Verbindung. Zur Beatmung wird somit das in der Befeuchterkammer 16 bevorratete, konditionierte Beatmungsgas aufgrund des vom Ventilator 22 erzeugten Überdrucks über den Beatmungsschlauch 13 hin zum Patienten befördert. Vom Patienten wird das Beatmungsgas eingeatmet und über die Beatmungsmaske wieder ausgeatmet. Der Patient bekommt hierbei eine Menge an Beatmungsgas zugeführt, die größer ist als die vom Patienten bei der Atmung verbrauchte Menge. Der Rest der vom Beatmungsgas zur Verfügung gestellten Beatmungsgasmenge verlässt den Beatmungskreis über das Leckagenventil 1. Bei der Inspiration durchströmt das Beatmungsgas das Innenlumen des Leckagenventils und wäscht hierbei sogleich auch alle Reste an verbrauchter Luft aus dem Leckagenventil heraus.

Bei der Handhabung kann es häufig vorkommen, dass sich der Beatmungsschlauch 13 zur am Patienten befindlichen Beatmungseinrichtung, z.B. Beatmungsmaske verdreht, wodurch Torsionskräfte entstehen und auf das Leckagenventil 1 wirken.

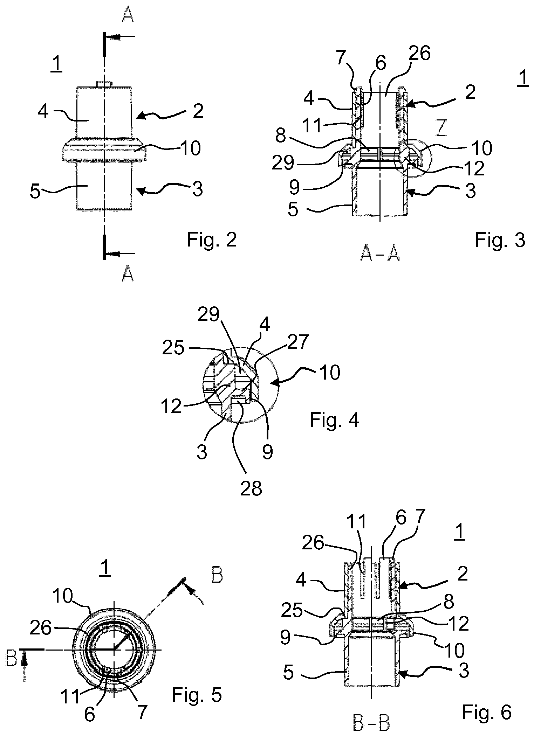

Fig. 2 zeigt ein Ausführungsbeispiel eines erfindungsgemäßen Leckagenventils. Das Leckagenventil 1 umfasst ein erstes rundes Gehäuseteil 2 sowie zweites rundes Gehäuseteil 3, wobei am ersten Gehäuseteil 2 ein erster Anschlussstutzen 4 und am zweiten Gehäuseteil 3 ein zweiter Anschlussstutzen 5 vorgesehen ist. Mit dem Anschlussstutzen 4 wird das Leckagenventil 1 mit der Beatmungseinrichtung 15, z.B. einer Beatmungsmaske, verbunden. Der zweite Anschlussstutzen 5 dient dazu, das Leckagenventil 1 mit dem Konnektor 24 mit Sondenanschlussmittel oder direkt mit dem Beatmungsschlauch 13 zu verbinden. Etwa im Mittelbereich des Leckagenventils 1 befindet sich ein sich erweiternder Gehäusebereich 10, in dem sich ein umlaufender schmaler Leckagenkanal 9, vgl. Fig. 3, für den Austritt der Leckagenströmung aus dem Leckagenventil 1 ins Freie befindet.

In Fig. 3 ist der konstruktive Aufbau des Leckagenventils 1 ersichtlich. Das zweite Gehäuseteil 3 bildet das Innenlumen des Leckagenventils 1, wobei das erste Gehäuseteil 2 an der Außenseite des zweiten Gehäuseteils 3 angeordnet und über eine Schnappverbindung in axialer Richtung verrastet ist. Das erste Gehäuseteil 2 wird hierzu über den zylindrischen Bereich des zweiten Gehäuseteils 3 geschoben. Der zylindrische Bereich weist gekrümmte Wandbereiche 26 auf, zwischen denen Schlitze 11 vorgesehen sind. Durch diese Schlitze 11 werden Rastlaschen 6 mit oberseitigen Nasen 7 gebildet, die mit der oberen Stirnseite des ersten Gehäuseteils 2 verrasten und letzteres somit in einer axialen Halteposition fixieren. Zwischen der Innenwand des ersten Gehäuseteils 2 sowie der Außenseite des zweiten Gehäuseteils 3 ist im Bereich des sich erweiterten Gehäusebereichs 10 ein ringförmiger Leckagenkanal 9 vorgesehen, über den Beatmungsgas ins Freie gelangen kann.

Des Weiteren sind am zweiten Gehäuseteil 3 radial nach außen gerichtete Stege 12 entlang des Umfangs des zweiten Gehäuseteils 3 vorgesehen, welche Durchgangsöffnungen 8 begrenzen. Über die Durchgangsöffnungen 8 kann Beatmungsgas aus dem Innenlumen des Leckagenventils in das Innere des erweiterten Bereichs 10 und von dort in den umlaufenden Leckagenkanal 9 gelangen. Die Durchgangsöffnungen 8 sind großflächig, da sie lediglich von den radial verlaufenden Stegen 12 begrenzt werden.

Im Bodenbereich der Stege 12 ist eine ringartige Erweiterung 27 vorgesehen, deren außenumfängliche Stirnfläche zusammen mit der Innenwandung des ersten Gehäuseteils 2 im Bereich der Gehäuseerweiterung 10 den Leckagenkanal 9 bildet.

Das erste Gehäuseteil 2 weist eine vorzugsweise umlaufende Stufenfläche 25 auf, die im verrasteten Zustand der beiden Gehäuseteile 2, 3 oberseitig auf den Stegen 12 aufliegt.

Die Konzeption ist noch etwas deutlicher in Fig. 4 ersichtlich. Die ringartige Erweiterung 27 besitzt an deren Unterseite eine umlaufende Ausnehmung 28, die zu einer mechanischen Versteifung des zweiten Gehäuseteils 3 bzw. der ringartigen Erweiterung 27 führt. Durch diese mechanische Stabilität wird verhindert, dass der Leckagenkanal 9 bei mechanischer Beanspruchung sich verändert und damit die Leckagenströmung beeinträchtigt wird.

Die ohne Kraftaufwand durchführbare Verdrehbarkeit der beiden Gehäuseteile 2, 3 im verrasteten Zustand zueinander wird dadurch realisiert, dass zwischen der axial verlaufenden Außenwand des ersten Gehäuseteils 2 im Bereich der Rastlaschen 6 und der Innenwand des ersten Anschlussstutzens 4 ein geringfügiges Spaltspiel vorgesehen ist. Ein entsprechendes Spaltspiel kann auch zwischen der stirnseitigen Nase 7 der Rastlaschen 6 und dem stirnseitigen Ende des ersten Anschlussstutzens 4 vorgesehen sein. Ebenfalls kann ein geringfügiges Spaltspiel auch zwischen der axial verlaufenden Außenwand, der gekrümmten Wandbereiche 26 des ersten Gehäuseteils 2 und der axial verlaufenden Innenwand der ersten Anschlussstutzens 4 vorgesehen sein.

Das Maß des Spalts (Spaltspiel) für die Drehbarkeit der beiden Gehäuseteile beträgt im Wesentlichen 0,08 mm bis 0,12 mm (d.h. auf den Durchmesser bezogen 0,04 mm bis 0,06 mm pro Seite), vorzugsweise 0,09 mm bis 0,11 mm (d.h. auf den Durchmesser bezogen 0,045 mm bis 0,055 mm pro Seite). Beispielsweise beträgt das Spaltspiel auf den Durchmesser bezogen 0,10 mm, d.h. 0,05 mm pro Seite.

Fig. 5 zeigt das Leckagenventil 1 in einer Ansicht von oben. Man erkennt die beiden gegenüberliegenden Rastlaschen 6 mit den oberseitigen stirnseitigen Nasen 7 des ersten Gehäuseteils 2, die das oberseitige Ende des zweiten Gehäuseteils 3 übergreifen.

Wie aus Fig. 6 deutlich wird, ist der oberseitige Bereich des ersten Gehäuseteils 2, d.h. der gekrümmte Wandbereich 26, bis zur Oberseite der Stege 12 glatt, d.h. ohne Stufe ausgebildet. Gleiches gilt für den außen angeordneten entsprechenden Bereich des ersten Gehäuseteils 2, der den ersten Anschlussstutzen 4 bildet.

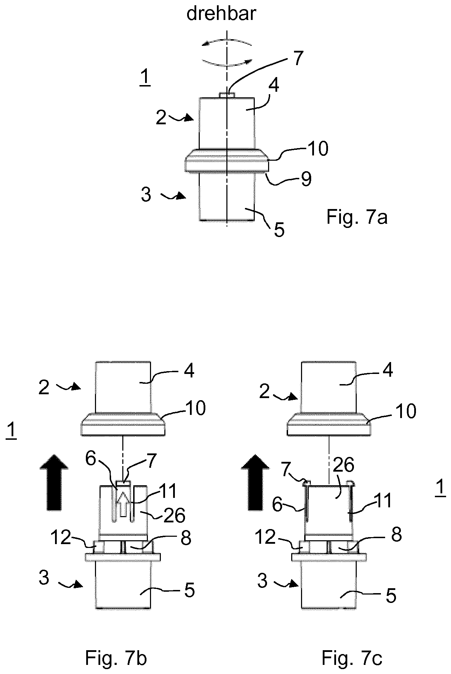

Wie aus Fig. 7a ersichtlich, können die beiden Gehäuseteile 2 und 3 des erfindungsgemäßen Leckagenventils 1 im verrasteten Zustand gegeneinander sehr leicht verdreht werden. Es können sich somit keine Torsionskräfte auf das Gehäuse des Leckagenventils 1 aufbauen, wenn z.B. der Beatmungsschlauch 13 verdreht wird.

Die Fig. 7b sowie 7c zeigen das Leckagenventil in einer Explosionsdarstellung. Gut erkennbar ist der jeweilige Bereich der entlang des Umfangs vorgesehenen Durchgangsöffnungen 8, durch die hindurch Atemgas aus dem Innenlumen des Leckagenventils 1 in den Ringraum 29 und von dort in den ringförmigen Leckagenkanal 9 (vgl. z.B. Fig. 3) gelangen kann.

Die erfindungsgemäße Konstruktion ermöglicht es zudem, die Gehäuseteile 2 sowie 3 einstückig aus Kunststoff in einem Spritzgießverfahren in einfacher Weise herzustellen.

Es wird ausdrücklich darauf hingewiesen, dass auch Unterkombinationen der beschriebenen Merkmalskomplexe sowie Ausführungsformen ausdrücklich vom Gegenstand der vorliegenden Erfindung als umfasst angesehen werden.

### BEZUGSZEICHENLISTE

- 1: Leckagenventil
- 2: erstes Gehäuseteil
- 3: zweites Gehäuseteil
- 4: erster Anschlussstutzen
- 5: zweiter Anschlussstutzen
- 6: Rastlasche
- 7: Nase
- 8: Durchgangsöffnung
- 9: Leckagenkanal
- 10: erweiterter Gehäusebereich
- 11: Schlitz
- 12: Steg
- 13: Beatmungsschlauch
- 14: Befeuchter
- 15: Beatmungseinrichtung
- 16: Befeuchterkammer
- 17: Wasserreservoir
- 18: Anschlussmuffe
- 19: Anschlussmuffe
- 20: Verbindungsschlauch
- 21: Verbinder
- 22: Ventilator
- 23: Konnektor mit Sondenanschlussmittel
- 24: Konnektor mit Sondenanschlussmittel
- 25: Stufenfläche
- 26: gekrümmter Wandbereich
- 27: ringartige Erweiterung
- 28: Ausnehmung
- 29: Ringraum

## Patentansprüche

1. Leckagenventil (1) für den Einsatz in einem Patientenbeatmungssystem mit
einem aus einem ersten (2) und zweiten Gehäuseteil (3) aufgebauten, vorzugsweise aus Kunststoff bestehenden Ventilgehäuse, wobei beide Gehäuseteile (2, 3) einen vorzugsweise im Querschnitt kreisförmigen Fluidkanal bilden, wobei das Ventilgehäuse folgendes umfasst:
einen ersten Anschlussstutzen (4) am ersten Gehäuseteil (2),
einen zweiten Anschlussstutzen (5) am zweiten Gehäuseteil (3) sowie einen radial nach außen vorspringenden Gehäusebereich (10) zwischen den beiden Anschlussstutzen (4, 5),
mindestens eine Durchgangsöffnung (8), die etwa in der Höhe des nach außen vorspringenden Gehäusebereichs (10) angeordnet ist und einen Austritt des Fluides aus dem Fluidkanal in Form einer Leckageströmung ermöglicht bzw. ermöglichen,
einen ebenfalls etwa in der Höhe des nach außen vorspringenden Gehäusebereichs (10) angeordneten, zumindest entlang eines Teils, vorzugsweise entlang des gesamten Umfangs des Ventilgehäuses umlaufenden, vorzugsweise durch beide Gehäuseteile (2, 3) gebildeten Leckagekanal (9) für den Austritt der Leckageströmung aus dem Leckageventil (1),
einen Verrastmechanismus, mittels dem die beiden Gehäuseteile (2, 3) miteinander verbindbar sind,
wobei als Verrastmechanismus mindestens eine, vorzugsweise eine Mehrzahl von entsprechend dem Innenradius des Anschlussstutzens (4 bzw. 5) gekrümmte(n) Rastlasche(n) (6) mit stirnseitiger Nase (7) vorgesehen sind, die im Zustand der Verrastung sich entlang des Anschlussstutzens (4 bzw. 5) innenseitig anliegend erstrecken und mit ihrer Nase (7) an der äußeren Stirnseite des Anschlussstutzens (4 bzw. 5) angreifen und eine axiale Verrastung gewährleisten und zwischen den Rastlaschen (6) entsprechend dem Innenradius des Anschlussstutzens (4 bzw. 5) gekrümmte Wandbereiche (26) vorgesehen sind, die an die Innenfläche des Anschlussstutzens (4 bzw. 5) angrenzen,wobei die beiden Gehäuseteile (2, 3) im verrasteten Zustand zueinander verdrehbar sind,
am zweiten Gehäuseteil (3) entlang des Umfangs mehrere radial sich erstreckende Stege (12) vorgesehen sind, zwischen denen die Durchgangsöffnungen (8) vorgesehen sind oder die die Durchgangsöffnungen (8) seitlich begrenzen,
die Stege (12) die gekrümmtem Wandbereiche (26) zur Außenseite hin überragen, und
zwischen der Innenwand des erweiterten Gehäusebereichs (10) und dem außenseitigen Ende des jeweiligen Stegs (12) ein umlaufender Ringraum (29) besteht.

2. Leckagenventil nach Anspruch 1, **dadurch gekennzeichnet, dass** im verrasteten Zustand der beiden Gehäuseteile (2, 3) zwischen der axial verlaufenden Außenwand der Rastlaschen (6) und der axial verlaufenden Innenwand des ersten Anschlussstutzens (4) ein Spaltspiel vorgesehen ist.

3. Leckagenventil nach Anspruch 1 oder 2, **dadurch gekennzeichnet, dass** im verrasteten Zustand der beiden Gehäuseteile (2, 3) zwischen der stirnseitigen Nase (7) der Rastlaschen (6) und dem stirnseitigen Ende des ersten Anschlussstutzens (4) ein Spaltspiel vorgesehen ist,

4. Leckagenventil nach den vorhergehenden Ansprüchen, **dadurch gekennzeichnet, dass** im verrasteten Zustand der beiden Gehäuseteile (2, 3) zwischen der axial verlaufenden Außenwand der gekrümmten Wandbereiche (26) und der axial verlaufenden Innenwand des ersten Anschlussstutzens (4) ein Spaltspiel vorgesehen ist.

5. Leckagenventil nach den Ansprüchen 1 bis 4, **dadurch gekennzeichnet, dass** im verrasteten Zustand der beiden Gehäuseteile (2, 3) der erste Gehäuseteil (2) auf den Stegen (12) aufliegt.

6. Leckagenventil nach den Ansprüchen 1 bis 5, **dadurch gekennzeichnet, dass** das erste Gehäuseteil (2) eine umlaufende Stufenfläche (25) aufweist, die im verrasteten Zustand der beiden Gehäuseteile (2, 3) oberseitig auf den Stegen (12) aufliegt.

7. Leckagenventil nach den vorhergehenden Ansprüchen, **dadurch gekennzeichnet, dass** das erste (2) und/oder zweite Gehäuseteil (3) einteilig ausgebildet sind.

8. Leckagenventil nach den vorhergehenden Ansprüchen, **dadurch gekennzeichnet, dass** das zweite Gehäuseteil (3) im Bereich des erweiterten Gehäusebereichs (10) eine ringartige Erweiterung (27) aufweist.

9. Leckagenventil nach Anspruch 8, **dadurch gekennzeichnet, dass** die ringartige Erweiterung (27) an deren Unterseite mit einer umlaufenden Ausnehmung (28) versehen ist.

10. Beatmungsschlauchsystem zur Verwendung bei einer Leckagenbeatmung umfassend einen, vorzugsweise beheizbaren, Beatmungsschlauch (13) sowie ein Leckagenventil (1) gemäß den vorherigen Ansprüchen.

11. Beatmungsschlauchsystem nach Anspruch 10, **dadurch gekennzeichnet, dass** an mindestens einem Ende, vorzugsweise an beiden Enden des Beatmungsschlauchs (13) eine Anschlussmuffe (18, 19) zum Anschluss des Beatmungsschlauchs (13) an das Leckagenventil (1) und/oder ein Funktionsteil vorgesehen ist.

12. Beatmungsschlauchsystem nach den Ansprüchen 10 bis 11, **dadurch gekennzeichnet, dass** sich das Leckagenventil (1) zwischen Anschlussmuffe (18) und einer am Patienten positionierten Beatmungseinrichtung (15) befindet.

13. Beatmungsschlauchsystem nach den Ansprüchen 10 bis 12, **dadurch gekennzeichnet, dass** sich das Leckagenventil (1) zwischen Anschlussmuffe (18) und einem Konnektor (24) mit Sondenanschlussmittel befindet.

## Claims

1. A leakage valve (1) for use in a patient ventilation system, comprising
a valve housing consisting of a first (2) and a second housing part (3) and preferably made of plastics material, wherein the two housing parts (2, 3) form a preferably circular fluid channel in cross-section, wherein the valve housing comprises the following:
a first connection piece (4) on the first housing part (2),
a second connection piece (5) on the second housing part (3) and a radially outwardly projecting housing region (10) between the two connection pieces (4, 5),
at least one through-opening (8), which is arranged approximately at the height of the outwardly projecting housing region (10) and allows or allow for the discharge of a fluid out of the fluid channel in the form of a leakage flow,
a leakage channel (9) for discharging the leakage flow out of the leakage valve (1), which leakage channel is also arranged approximately at the height of the outwardly projecting housing region (10), which extends around at least some and preferably the entire circumference of the valve housing, and which is preferably formed by means of the two housing parts (2, 3),
a locking mechanism, by means of which the two housing parts (2, 3) can be connected to one another,
wherein at least one, preferably multiple latching tab(s) (6) are provided as the locking mechanism, which are curved according to the inner radius of the connection piece (4 or 5), and which comprise a front nose (7), and which, in the locking state, extend along the inside of the connection piece (4 or 5) and engage with their nose (7) in the outer front face of the connection piece (4 or 5) and ensure axial locking, and wall regions (26) are provided between the latching tabs (6), which wall regions are curved according to the inner radius of the connection piece (4 or 5) and which adjoin the inner surface of the connection piece (4 or 5), wherein the two housing parts (2, 3) can be rotated relative to one another in the locked state,
multiple radially extending webs (12) are provided on the second housing part (3) around the circumference, between which webs the through-openings (8) are provided or which webs laterally delimit the through-openings (8),
the webs (12) project outwards beyond the curved wall regions (26), and
there is a circumferential annular space (29) between the inner wall of the extended housing region (10) and the outer end of the relevant web (12).

2. The leakage valve according to claim 1, **characterized in that**, in the locked state of the two housing parts (2, 3), a gap clearance is provided between the axially extending outer wall of the latching tabs (6) and the axially extending inner wall of the first connection piece (4).

3. The leakage valve according to claim 1 or 2, **characterized in that**, in the locked state of the two housing parts (2, 3), a gap clearance is provided between the front nose (7) of the latching tabs (6) and the front end of the first connection piece (4).

4. The leakage valve according to the preceding claims, **characterized in that**, in the locked state of the two housing parts (2, 3), a gap clearance is provided between the axially extending outer wall of the curved wall regions (26) and the axially extending inner wall of the first connection piece (4).

5. The leakage valve according to claims 1 to 4, **characterized in that**, in the locked state of the two housing parts (2, 3), the first housing part (2) rests on the webs (12).

6. The leakage valve according to claims 1 to 5, **characterized in that** the first housing part (2) comprises a circumferential step surface (25), which rests on the webs (12) in the locked state of the two housing parts (2, 3).

7. The leakage valve according to the preceding claims, **characterized in that** the first (2) and/or second housing part (3) are formed in one piece.

8. The leakage valve according to the preceding claims, **characterized in that** the second housing part (3) comprises a ring-like extension (27) in the region of the extended housing region (10).

9. The leakage valve according to claim 8, **characterized in that** the ring-like extension (27) is provided with a circumferential recess (28) on its underside.

10. A ventilation tube system for use in leak ventilation, comprising a, preferably heatable, ventilation tube (13) as well as a leakage valve (1) according to the preceding claims.

11. The ventilation tube system according to claim 10, **characterized in that** a connection sleeve (18, 19) for connecting the ventilation tube (13) to the leakage valve (1) and/or a functional component is provided on at least one end, preferably on both ends of the ventilation tube (13).

12. The ventilation tube system according to claims 10 to 11, **characterized in that** the leakage valve (1) is located between the connection sleeve (18) and a ventilator (15) positioned by the patient.

13. The ventilation tube system according to claims 10 to 12, **characterized in that** the leakage valve (1) is located between the connection sleeve (18) and a connector (24) comprising probe connection means.

## Revendications

1. Valve à fuite intentionnelle (1) destinée à être utilisée dans un système de respiration avec
un boîtier de valve constitué d'une première (2) et d'une deuxième partie de boîtier (3), de préférence de plastique, les deux parties de boîtier (2, 3) formant un canal de fluide dont la section transversale est de préférence circulaire, le boîtier de valve comportant :
une première tubulure de raccordement (4) sur la première partie de boîtier (2),
une deuxième tubulure de raccordement (5) sur la deuxième partie de boîtier (3) ainsi qu'une région de boîtier faisant saillie radialement vers l'extérieur (10) entre les deux tubulures de raccordement (4, 5),
au moins une ouverture de passage (8) disposée approximativement à la hauteur de la région de boîtier faisant saillie radialement vers l'extérieur (10) et permettant une sortie du fluide hors du canal de fluide sous la forme d'un courant de fuite,
un canal de fuite (9) également disposé à la hauteur de la région de boîtier faisant saillie radialement vers l'extérieur (10), s'étendant au moins le long d'une partie de la circonférence du boîtier de valve, de préférence le long de la totalité de celle-ci, formé de préférence par les deux parties de boîtier (2, 3) pour la sortie du courant de fuite hors de la valve à fuite intentionnelle (1),
un mécanisme d'encliquetage, au moyen duquel les deux parties de boîtier (2, 3) peuvent être reliées entre elles,
dans laquelle, en tant que mécanisme d'encliquetage, il est prévu au moins une, de préférence une pluralité de patte(s) d'encliquetage (6) incurvée(s) de manière correspondante au rayon intérieur de la tubulure de raccordement (4 ou 5) avec un nez côté frontal (7), laquelle/lesquelles s'étend(-ent) le long de la tubulure de raccordement (4 ou 5) en s'appliquant sur le côté intérieur de celle-ci dans l'état de l'encliquetage tout en engageant le côté frontal extérieur de la tubulure de raccordement (4 ou 5) avec leur nez (7) et en assurant un encliquetage axial, et il est prévu des régions de paroi incurvées (26) de manière correspondante au rayon intérieur de la tubulure de raccordement (4 ou 5) entre les pattes d'encliquetage (6), lesquelles sont adjacentes à la surface intérieure de la tubulure de raccordement (4 ou 5), dans laquelle les deux parties de boîtier (2, 3) peuvent être tournées l'une vers l'autre dans l'état encliqueté,
sur la deuxième partie de boîtier (3), il est prévu plusieurs âmes (12) s'étendant radialement le long de la circonférence, entre lesquelles sont prévues les ouvertures de passage (8) ou lesquelles délimitent les ouvertures de passage (8) latéralement,
les âmes (12) font saillie au-delà du côté extérieur des régions de paroi incurvées (26), et
entre la paroi intérieure de la région de boîtier élargie (10) et l'extrémité côté extérieur de l'âme (12) respective subsiste un espace annulaire circonférentiel (29).

2. Valve à fuite intentionnelle selon la revendication 1, **caractérisée en ce que** dans l'état encliqueté des deux parties de boîtier (2, 3), il est prévu un jeu de fente entre la paroi extérieure s'étendant axialement des pattes d'encliquetage (6) et la paroi intérieure s'étendant axialement de la première tubulure de raccordement (4).

3. Valve à fuite intentionnelle selon la revendication 1 ou 2, **caractérisée en ce que** dans l'état encliqueté des deux parties de boîtier (2, 3), il est prévu un jeu de fente entre le nez côté frontal (7) des pattes d'encliquetage (6) et l'extrémité côté frontal de la première tubulure de raccordement (4),

4. Valve à fuite intentionnelle selon les revendications précédentes, **caractérisée en ce que** dans l'état encliqueté des deux parties de boîtier (2, 3), il est prévu un jeu de fente entre la paroi extérieure s'étendant axialement des régions de paroi incurvées (26) et la paroi intérieure s'étendant axialement de la première tubulure de raccordement (4).

5. Valve à fuite intentionnelle selon les revendications 1 à 4, **caractérisée en ce que** dans l'état encliqueté des deux parties de boîtier (2, 3), la première partie de boîtier (2) s'appuie sur les âmes (12).

6. Valve à fuite intentionnelle selon les revendications 1 à 5, **caractérisée en ce que** la première partie de boîtier (2) présente une surface étagée circonférentielle (25), laquelle s'appuie sur les âmes (12) côté supérieur dans l'état encliqueté des deux parties de boîtier (2, 3).

7. Valve à fuite intentionnelle selon les revendications précédentes, **caractérisée en ce que** la première (2) et/ou la deuxième partie de boîtier (3) sont réalisées en une seule pièce.

8. Valve à fuite intentionnelle selon les revendications précédentes, **caractérisée en ce que** la deuxième partie de boîtier (3) présente un élargissement du genre anneau (27) dans la région de la région de boîtier élargie (10).

9. Valve à fuite intentionnelle selon la revendication 8, **caractérisée en ce que** l'élargissement du genre anneau (27) est pourvu d'un évidement circonférentiel (28) sur son côté inférieur.

10. Système de tube respiratoire destiné à être utilisé dans le cadre d'une ventilation à fuite, comportant un tube respiratoire (13) de préférence chauffable ainsi qu'une valve à fuite intentionnelle (1) selon les revendications précédentes.

11. Système de tube respiratoire selon la revendication 10, **caractérisé en ce qu'**à au moins une extrémité, de préférence aux deux extrémités du tube respiratoire (13), il est prévu un manchon de raccordement (18, 19) permettant de raccorder le tube respiratoire (13) à la valve à fuite intentionnelle (1) et/ou une partie fonctionnelle.

12. Système de tube respiratoire selon les revendications 10 à 11, **caractérisé en ce que** la valve à fuite intentionnelle (1) se trouve entre le manchon de raccordement (18) et un dispositif respiratoire (15) positionné sur le patient.

13. Système de tube respiratoire selon les revendications 10 à 12, **caractérisé en ce que** la valve à fuite intentionnelle (1) se trouve entre le manchon de raccordement (18) et un connecteur (24) comprenant un moyen de raccordement de sonde.
